# EUROPEAN PATENT SPECIFICATION

(11) **EP 0 711 609 B1**
(45) Date of publication and mention of the grant of the patent: **09.02.2000**
(21) Application number: 95307936.5
(22) Date of filing: 07.11.1995
(51) Int. Cl.: B05B 7/00, A61M 11/06

(54) **Atomizer**
Zerstäuber
Atomiseur

(30) Priority: 11.11.1994 GB 9422821
(43) Date of publication of application: 15.05.1996
(73) Proprietor: Medic-Aid Limited, Southampton SO14 2PT (GB)
(72) Inventor: Denyer, Jonathan S., Pagham, West Sussex, PO21 3LQ (GB)
(74) Representative: Arthur, Bryan Edward

(56) References cited:
- FR-A- 1 070 292

## Description

The present invention relates to atomizers and, in particular, to atomizers of the type which include a gas exit, at least one outlet in the region of the gas exit and a deflector for deflecting gas issuing from the gas exit across the at least one outlet whereby a substance to be atomized is drawn out of the at least one outlet and atomized. These atomizers atomize liquids or powders into the gas.

Most conventional atomizers of the above type operate continuously whether atomization is required or not. Strictly speaking, when such atomizers, frequently called nebulisers, are used in medical applications, atomization is only required during the inhalation phase of a breathing cycle so that a drug can be administered by deposition in the lungs. In practice a patient usually inhales for about 30 percent of the breathing cycle, consequently, use of a continuously operating atomizer results in a large proportion of the atomized drug being wasted.

Some designs of medical atomizer overcome such wastage by giving the patient a trigger to start the atomization when they begin to inhale. Such a trigger controlled type of atomizer is not satisfactory since the patient must coordinate inhalation with trigger operation.

In one conventional atomizer a gas duct leads gas under pressure to a gas exit, a reservoir for holding the substance to be atomized is formed around the base of the gas duct, and a sleeve placed around the gas duct defines a passageway through which the substance to be atomised may pass to at least one outlet. A fixed deflector in the form of a bar is disposed in line with the gas outlet so that gas issuing from the gas exit is deflected so as to pass over the outlet or outlets. The passage of gas over each outlet draws the substance to be atomised from the reservoir, through the passageway to each outlet. The deflected gas atomises the substance, and atomised particles of the substance are carried away during the inhalation phase of the patient since the patient breathes air or gas in through the atomiser some of the drug is lost while the patient is not inhaling.

FR-A-1070292 discloses an atomiser having a nozzle with an opening outlet through which air is directed, and a tube having an outlet from which a liquid is drawn by the air exiting the air outlet. In use, the liquid is drawn up the tube by the air leaving the nozzle and a spray of the liquid is generated. A deflector is movable into and out of the trajectory of the spray in order to allow the substance to be dispensed in two different ways, one allowing for a fine aerosol for inhalation and the other for a coarse spray of wetting particles.

Atomisers are used in other applications. For example, powders or liquid may be sprayed from a jet, the liquid or powder being atomised and entrained by a propellant. In conventional sprays, operation is controlled by a valve for releasing propellant. When the valve is released, the spraying operation is stopped and some of the liquid or powder collects in the jet since insufficient propellant has been released. The collected spray either dries to block the jet or is propelled by a re-started spraying operation in large droplets. Where paint is being sprayed, this causes splatter and uneven deposition on a surface to be painted.

It is an object of this invention to reduce at least some of the above disadvantages of the above-mentioned prior art.

The present invention is defined in the appended claims.

Embodiments of the invention are described below by way of example only with reference to the accompanying drawings in which:
Figure 1 shows a first embodiment of the invention in a relaxed position;
Figure 2 shows the first embodiment of the invention in a first operational position in which atomization takes place;
Figure 3 shows the first embodiment of he invention in a second operational position in which atomization takes place;
Figure 4 shows the first embodiment of the invention in a third operational position in which no atomization takes place;
Figures 5 and 6 show one embodiment of the flap valve and gas deflector;
Figure 7 shows a second embodiment of the invention in a second operational position in which atomization takes place,
Figure 8 shows a further embodiment of the invention in which the movable baffle bar is located beneath the baffle, and
Figure 9 shows the further embodiment during inhalation by a patient.

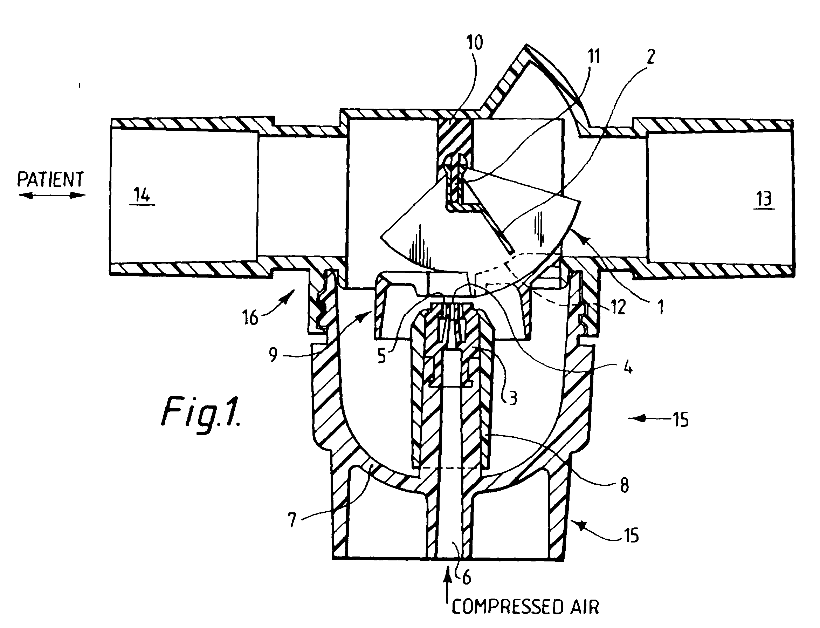

Referring to Figures 1 to 4, an atomizer includes a gas duct 6 which leads gas under pressure to a gas exit 4 within a jet head 3. The gas duct 6 passes through a wall of a reservoir 7 within which a substance to be atomised is held. A sleeve 8 is disposed around the jet head 3 and the gas duct 6. Passages are formed between the inner surface of the sleeve 8 and the outer surface of the gas duct 6 for leading the substance to be atomized from the reservoir 7 to outlets 5 in the jet head adjacent to the gas exit 4. For atomization of the substance to take place, a deflector 1 must be placed in the path of the pressurised gas exiting from the gas exit 4 so that it is redirected to pass directly over the outlets 5. This flow of pressure air draws the substance to be atomized from the reservoir 7, through the passage between the sleeve 8 and the gas duct 6 to the outlets 5. The flow of pressure air atomizes the substance as the substance leaves the outlets 5.

A downwardly and outwardly shaped baffle 9 is disposed around the jet head 3 to the atomized substance downwards before it is carried away. It is important that the substance is atomized into very fine droplets. In medical applications, the substance to be atomized is a drug for administering to a patient by lung deposition. The finer the droplets, the deeper into the lungs the drug will pass. This maximises the deposition of the drug. Larger droplets collect on the inside of the baffle 9 where they coalesce to drop back down into the reservoir 7.

The atomizer also includes an air inlet 13 and an air outlet 14. In the above-mentioned medical application, as a patient inhales, ambient air is drawn into the atomizer through the inlet 13. The air then passes into the region of the air exit 4 and outlets 5 where droplets are entrained by the inhaled ambient air. The air then passes down under the baffle 9 before passing upwardly and out via the air outlet 14 carrying droplets of the drug to the patient. This action is described in more detail in our British Patent applications 9219327.5 and 9311614.3 which are hereby imported into this description in their entirety.

A planar arcuate gas deflector 1 is mounted above the gas exit to be movable about a pivot in that plane. The gas deflector 1 may be disposed across the gas exit 4, in which case atomization takes place, or may be disposed away from the gas exit 4, in which case no atomization takes place.

A vane or flap 2 is joined to the deflector bar 1 so as to be pivotally mounted and to move with the deflector bar 1. The flap 2 responds to the breathing pattern of a patient by moving around the pivot.

When the apparatus is not in use, the vane assumes the position shown in Figure 1 in which the gas deflector is not disposed across the gas exit. The vane forms a partial seal against a curved surface 12 (shown in outline). Even when pressure gas is issuing from the gas exit, no atomization takes place since the deflector is not disposed in the path of the gas.

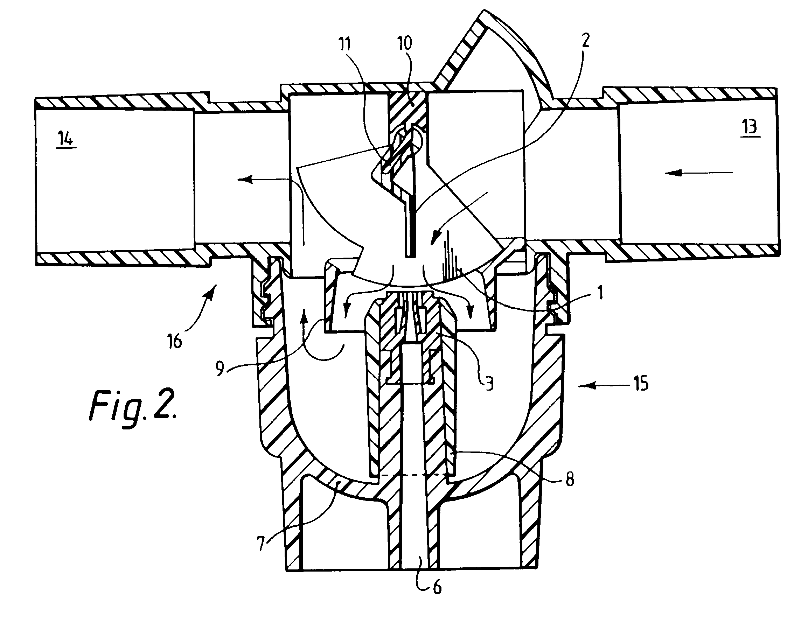

When a patient inhales, ambient air is drawn into the atomizer through the air inlet 13. The vane 2 is displaced into the position shown in Figure 2 permitting and directing the ambient air to pass into the region of the gas exit before being directed downwardly and outwardly around the baffle. The air then escapes via the air outlet 14 to the patient. The displacement of the flap moves the deflector bar into the path of the gas issuing from the gas exit. Atomization therefore begins as soon as the patient begins to breath in. The atomized drug is carried away by the air passing through the atomizer. The vane must move only a few degrees before the deflector bar 1 is brought into position to commence atomization, but must move a few more degrees before breaking the seal between the flap and the curved surface to permit ambient air to enter the atomizer. This ensures that the deflector is fully in position and atomizing cleanly before the ambient air passes through the atomizer to carry the droplets away.

The deflector extends further from the pivot than the flap so that the deflector can be positioned very close to the gas exit without obstruction from the flap. The curved surface 12 against which the vane seals therefore includes an arcuate slot through which the deflector may pass.

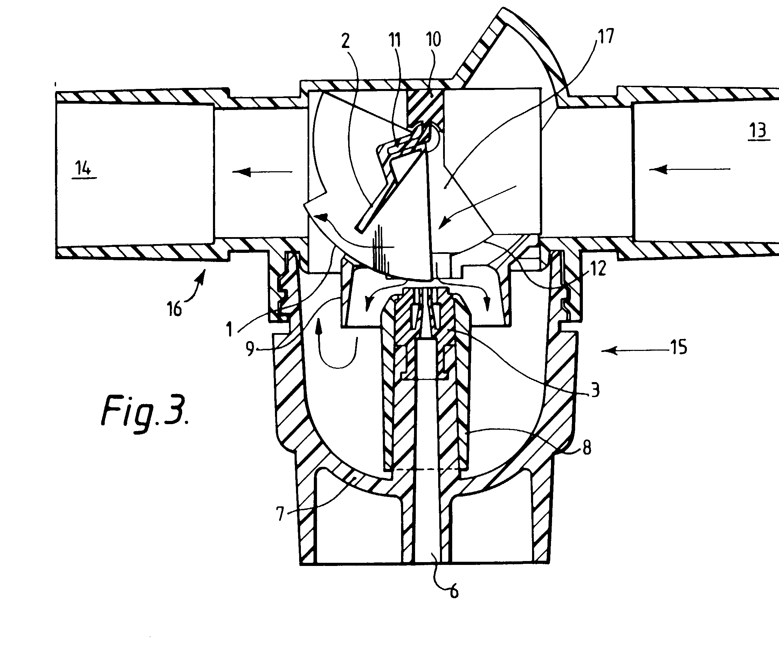

If the patient inhales sharply or quickly, the vane 2 assumes the position shown in Figure 3 wherein the deflector bar 1 remains in the path of the gas exit so that atomization takes place, but excess air passes directly from the air inlet 13 to the air outlet 14 without entraining the atomized substance. The main reason for this is that the efficiency of entrainment of droplets decreases where air passes through the atomizer too quickly since a proportion of droplets will impact against the walls of the atomizer. A typical optimum flow rate is of the order of twenty five litres per minute.

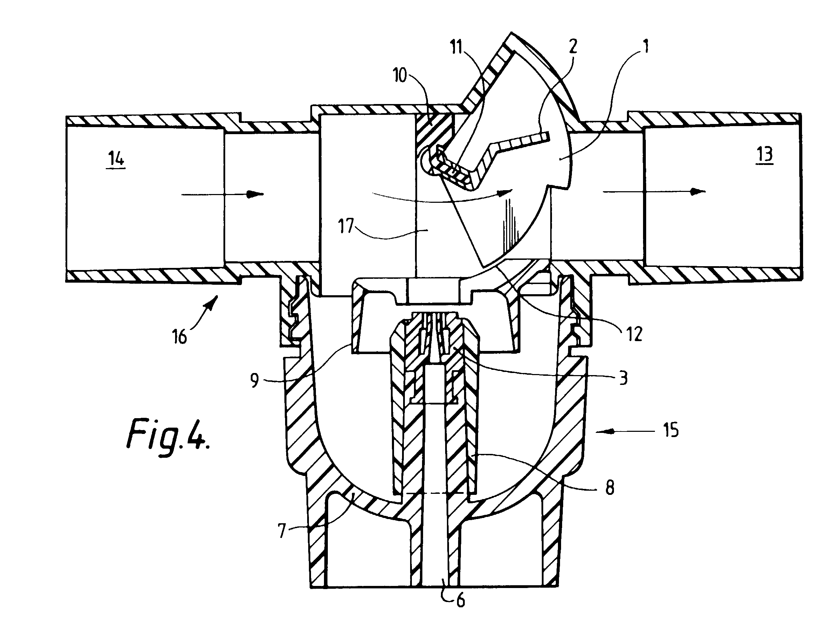

When the patient exhales, the vane 2 is displaced to a position as shown in figure 4 where the deflector is displaced such that it is not in the path of the gas exit. Atomization therefore does not occur, and so no drug is wasted. The vane allows exhaled air to pass directly from the air outlet 14 to the air inlet 13 without having to pass through the atomizing chamber. The combination of the vane 2 and the deflector 1 therefore constitutes a one-way valve.

When the patient is not breathing in or out, the vane 2 is biassed towards the position shown in Figure 1. The vane 2 and deflector 1 are mounted on a rubber tongue 11 extending from a fixed rubber block 10. The vane 2 and deflector 1 are therefore resiliently mounted.

The atomizer shown in Figures 1 to 4 includes three separable units. A base unit 15 includes the reservoir 7, the gas duct 6, the jet head 3 and outlets 5. The reservoir 7 includes a threaded rim. An upper unit 16 includes three air inlet 13, and the air outlet 14. The baffle 9, sleeve 8, frame members 17, the vane 2, gas deflector 1, the rubber tongue 11 and the fixed rubber block 10 constitute the third unit. Separation of the third unit permits the atomizer to be more easily cleaned. The vane 2 and gas deflector are connected-to to the air inlet 13 in the upper unit 16, and to the baffle 9 since the gas deflector 1 must pass through a slot in the baffle. The sleeve 8 may be part of the base unit 15, or part of the third unit.

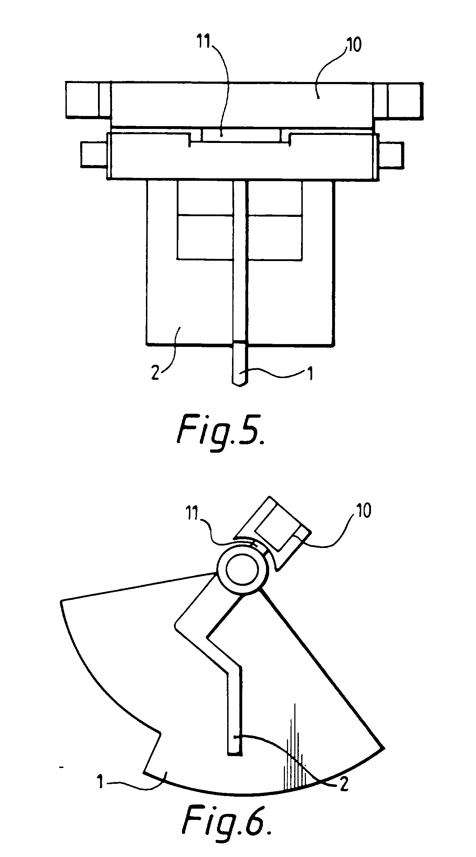

Figures 5 and 6 show the vane 2 and deflector 1 mounted on the fixed rubber block and rubber tongue. The rubber tongue 11 is held at the ends by the frame members 17 so that when the tongue 11 is bent by the vane, a load is applied. The vane 2 and deflector 1 are attached directly to the tongue 11 so that they are pivotally displaceable.

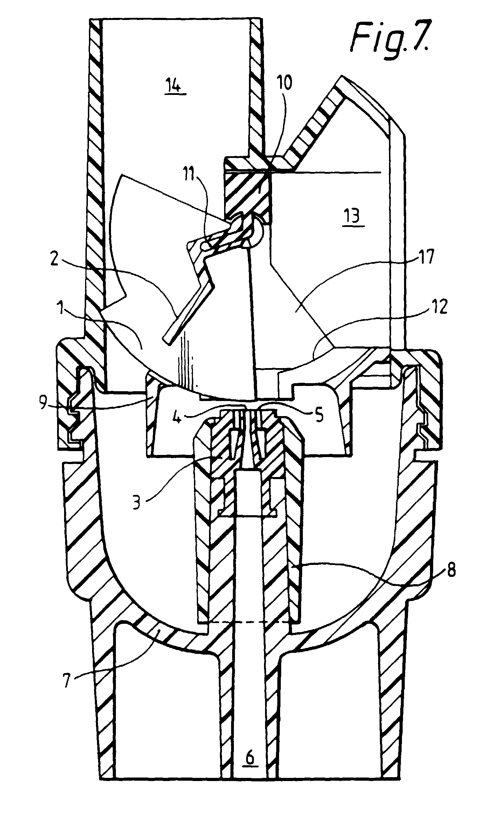

Figure 7 shows a second embodiment of this invention in which the base unit 15 is exactly as described in relation to Figures 1 to 4. The vane 2 and deflector 1 are also mounted as described above. The main difference in this second embodiment is that the air outlet 14 leading to a patient extends vertically from the atomizer as shown in Figure 7. Drug laden air does not have to pass around a sharp corner into the air outlet 14 once it has passed around the baffle 9. Fewer drug droplets will collect on the inner surface of the air outlet 14.

Referring to Figures 3, 4 and 7, the lower edge of the vane 2 forms a seal with the curved surface 12 as explained above. The vane 2 also includes two other edges which must be sealed. The vane 2 swings between two vertical wedgeshaped frame members 17 which form a seal so that flow of air to bypass the vane 2 is restricted when the vane 2 is disposed in any of the positions shown in Figures 1, 2 or 7. In the first and second embodiments shown in Figures 1 to 7, the frame members 17 also act as supports for the fixed rubber block 10. The frame members 17 may extend from the baffle 9, from the edge of the curved surface 12 or from the base unit 15. Where the frame members extend from the edge of the curved surface 12, the vane 2, deflector 1 and rubber block 10 are all mounted on the frame members 17 and within the upper unit 16.

According to another embodiment (not shown), the rubber block 10 is replaced by a metal spring eg, a leaf spring which permits the vane 2 and deflector 1 to be pivotally moveable in the same manner as described in relation to the rubber block 10 and tongue 11.

In a further embodiment (not shown) the vane 2 is emitted, and the deflector is movable into and out of the stream of gas issuing from the gas exit according to the breathing pattern of a patient. The vane is replaced by a flow sensor which detects when a patient begins to inhale and moves the deflector 1 into the path of gas issuing from the gas exit. In this embodiment the deflector is a bar which is moveable perpendicularly or laterally relative to the longitudinal extent of the bar.

In another embodiment the deflector 1 is displaceable up and down in line with the gas issuing from the gas outlet. Once the deflector is raised above a certain height, atomization ceases to take place.

In yet a further embodiment, the deflector is not a straight bar, but is of any suitable shape for deflecting the gas across the outlets to cause atomization. The deflector-may, for example, be a spherical ball disposed in the path of gas exiting the gas exit. The deflector may be a longitudinal blade movable into the path of the gas in the longitudinal direction of the blade.

In yet another embodiment (not shown) of this invention, the atomizer is used for producing a spray. This spray may be liquid droplets or powder particles. In medical applications, the spray may contain a drug. This spray producing apparatus may be used for producing sprays of paint droplets, perfume droplets or any other suitable liquids or powders. A base unit 15 of Figures 1 - 4 may be used to produce a gas exit 4 and outlets 5 for the substance to be atomized. A moveable deflector 1 is displaceable by a user. The user first activates a compressor which sends gas through the gas duct. For paint spraying, a mechanical compressor may be used, although this could be substituted for an aerosol propellant. The user then moves the deflector into the path of the gas issuing from the gas exit 4 to start atomization. The propellant then carries the droplets or powder through an outlet jet to form a spray. The user stops atomization before stopping the flow of gas from the gas exit. This keeps the outlet jet clean and free from paint and the like. A two-stage button can be used whereby atomization only takes place when the button is fully depressed while gas issues from the gas exit when the button is only partially depressed.

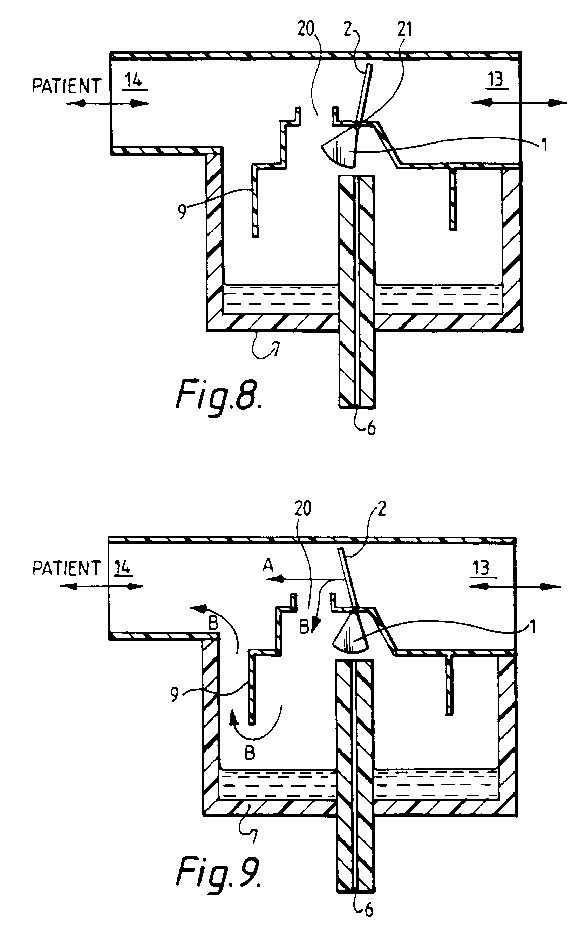

Under certain conditions, although 95% of the gas issuing from the gas exit 4 is deflected to either side of the deflector bar 1, a small amount hits the baffle bar depositing the substance to be atomized on the deflector bar 1. The gas which hits the baffle bar drives the liquid along the baffle bar towards the ends where the liquid can collect on top of the baffle 9 so that it is lost to the atomizer system. The whole dose of medicament is then not available to be administered to the patient. Furthermore, in some arrangements, as the deflector bar is moved out of the flow of gas issuing from the gas exit 4, the liquid that is running along the edge of the deflector bar 1 is sprayed into the top of the nebulizer where it collects without returning back to the reservoir 7. Referring now to Figures 8 and 9, the deflector bar 1 is housed entirely within the baffle 9 so that any liquid which collects on the deflector bar merely drips back into the reservoir, or if it is sprayed from the deflector bar by the flow of gas from the gas exit 4, is collected on the underside of the baffle 9 whereupon it coalesces and drops back down into the reservoir 7. Figure 8 shows the nebulizer in a position where the patient is not inhaling. The segment shaped deflector bar 1 is disposed outside the line of gas exiting from the gas exit 4 so that nebulization does not take place. The segment is pivoted at a pivot point 21, and is also connected to the vane or flap 2. When a patient inhales, air is drawn into the nebulizer through air inlet 13, and deflects the vane or flap 2 moving the deflector bar 1 into line with the gas exit thus causing atomization of the substance to occur. For clarity, the outlets 5 and the sleeve 8 are not shown in the Figure. However the jet head is arranged in the same way as described in connection with Figures 1 to 7. The atomization of the substance causes the pressure beneath the baffle 9 to be decreased thereby drawing part of the inhaled air under the baffle 9 as shown by arrow B. The baffle 9 includes an aperture 20 for permitting the flow of air for entraining droplets B to enter beneath the baffle 9. A proportion of the inhaled air passes directly from the air inlet 13 to the air outlet 14 as shown by arrow A. Once the flow of air for entraining droplets B has passed beneath the baffle 9, it returns around the outside of the baffle 9 to rejoin the through flow of air A. A further advantage of this embodiment is that only a certain volume of air passes under the baffle 9 in a given time. The nebulizer works most effectively when the flow of air for entraining droplets is of the rate of about 25 litres per minute. If this rate of flow of air is much greater than this or much less than this, the effectiveness of entrainment decreases. This means that if the patient inhales sharply, the rate of through flow of air A increases without significantly altering the flow of air for entraining droplets B passing beneath the baffle.

## Claims

1. An atomiser including a gas exit (4), an outlet (5) adjacent the gas exit (4), and a deflector (1) for deflecting gas issuing from the gas exit (4), wherein the deflector (1) is movable between a first position in the path of the gas issuing from the gas exit (4) and a second position, characterised in that the deflector (1) serves to deflect gas issuing from the gas exit (4) over the outlet (5) for drawing a substance to be atomised out from the outlet (5) and atomising the substance in the gas issuing from the gas exit (4), in that the first position of the deflector (1) is for atomisation, and in that the second position is a non-atomising position.

2. An atomiser according to claim 1, characterised by means for moving the deflector between the first and second positions.

3. An atomiser according to claim 2, characterised in that the means for moving the deflector is a vane (2) which is deflectable by the passage of air drawn through the atomiser.

4. An atomiser according to claim 3, characterised in that the vane (2) is hingedly mounted for pivotal movement.

5. An atomiser according to any one of the preceding claims, characterised in that the deflector (1) is a bar.

6. An atomiser according to any one of the preceding claims, including an air inlet (13) and an air outlet (14) for passage of air carrying the atomised substance to a patient, and wherein the deflector (1) moves into the first position for atomisation of the substance only during inspiration by a patient.

7. An atomiser according to any one of claims 1 to 5, characterised by an air inlet (13) and an air outlet (14) for the flow of air, the air outlet (14) serving to permit flow of the atomised substance to a patient.

8. An atomiser according to claim 7, characterised in that the deflector (1) moves into the first position for atomisation when the flow of air passes from the air inlet (13) to the air outlet (14).

9. An atomiser according to claim 7 or 8, characterised in that the deflector (1) moves into the second and non-atomising position when the flow of air is interrupted.

10. An atomiser according to claims 7 to 9, characterised in that the vane (2) is biassed to a position closing the air inlet (13).

11. An atomiser according to any one of claims 8 to 10, characterised in that, when the flow of air passes from the air inlet (13) to the air outlet (14), the vane (2) directs air into the region of the atomiser in which atomisation takes place.

12. An atomiser according to claims 10 and 11, characterised in that when the flow of air passes from the air outlet (14) to the air inlet (13), the vane (2) permits air to flow directly from the air outlet (14) to the air inlet (13) without passing through the region of the atomiser in which atomisation takes place when the deflector is in the first position.

13. An atomiser according to any one of claims 7 to 12, characterised in that when the flow of air from the air inlet (13) to the air outlet (14) exceeds a predetermined flow rate, a proportion of the air flow bypasses the region of the atomiser in which atomisation takes place when the deflector is in the first position.

14. An atomiser according to any preceding claim, characterised in that the deflector (1) is a longitudinal deflector, and is movable laterally with respect to its length between its first and second positions.

15. An atomiser according to any one of claims 1 to 13, characterised in that the deflector (1) is arcuate and angularly displaceable around the axis of that arc.

16. An atomiser according to any one of the preceding claims, characterised in that the deflector (1) includes a knife edge which is presented in the first position to the gas issuing from the gas exit (4).

17. An atomiser according to any one of claims 1 to 13, characterised in that the deflector (1) is arcuate and angularly displaceable around the axis of that arc, and includes a knife edge which is presented in the first position to the gas issuing from the gas exit (4), and characterised in that the deflector is moveable longitudinally with respect to its edge between the first and second positions.

18. An atomiser according to any one of the preceding claims, wherein the deflector, when moved into its second position, lies outside the path of the gas issuing from the gas exit.

19. An atomiser according to any one of claims 1 to 16, characterised in that the deflector is movable towards and away from the gas exit (4).

20. An atomiser according to claim 19, characterised in that the deflector, when moved into its second position is spaced away from the gas exit such that atomisation does not occur.

21. An atomiser according to any one of claims 6 to 20, characterised in that the flow of air is generated by the breathing pattern of a person.

22. An atomiser according to claim 21, characterised in that the atomised substance is entrained by air inhaled by the person.

23. An atomiser according to claim 21 or 22, characterised in that the air exhaled by the person does not entrain the substance to be atomised.

24. An atomiser according to any preceding claim, characterised in that the substance to be atomised is selected from: a liquid, a powdered solid, and a colloid.

25. An atomiser according to any preceding claim, characterised by a jet outlet for generating a spray of the substance to be atomised.

26. An atomiser according to claim 1 or 2, wherein the means for moving the deflector includes an air flow sensor and means responsive to the sensor for changing the position of the deflector.

27. An atomiser according to any one of claims 1 to 4, wherein the deflector is a ball.

28. An atomiser according to any preceding claim, further comprising a baffle (9) extending outwardly and downwardly about the gas exit (4), the deflector (1) being mounted within the baffle (9).

## Patentansprüche

1. Zerstäuber, umfassend einen Gasaustritt (4), einen dem Gasaustritt (4) benachbarten Auslaß (5) und eine Ablenkvorrichtung (1) zum Ablenken eines Gases, welches aus dem Gasaustritt (4) austritt, wobei die Ablenkvorrichtung (1) zwischen einer ersten Position in dem Weg des Gases, welches aus dem Gasaustritt (4) austritt, und einer zweiten Position beweglich ist, dadurch gekennzeichnet, daß die Ablenkvorrichtung (1) dazu dient, ein Gas, welches aus dem Gasaustritt (4) austritt, über den Auslaß (5) abzulenken, um eine zu zerstäubende Substanz aus dem Auslaß (5) zu saugen und die Substanz in dem Gas, welches aus dem Gasaustritt (4) austritt, zu zerstäuben, daß die erste Position der Ablenkvorrichtung (1) zur Zerstäubung dient und daß die zweite Position eine Nicht-Zerstäubungs-Position ist.

2. Zerstäuber nach Anspruch 1, gekennzeichnet durch eine Einrichtung zum Bewegen der Ablenkvorrichtung zwischen der ersten und zweiten Position.

3. Zerstäuber nach Anspruch 2, dadurch gekennzeichnet, daß die Einrichtung zum Bewegen der Ablenkvorrichtung ein Blatt (2) ist, welches durch den Durchfluß von Luft, welche durch den Zerstäuber gesaugt wird, ausgelenkt werden kann.

4. Zerstäuber nach Anspruch 3, dadurch gekennzeichnet, daß das Blatt (2) schwenkbar für eine Schwenkbewegung angebracht ist.

5. Zerstäuber nach einem der vorhergehenden Ansprüche, dadurch gekennzeichnet, daß die Ablenkvorrichtung (1) eine Stange ist.

6. Zerstäuber nach einem der vorhergehenden Ansprüche, umfassend einen Lufteinlaß (13) und einen Luftauslaß (14) zum Durchgang von Luft, welche die zerstäubte Substanz zu einem Patienten trägt, und wobei sich die Ablenkvorrichtung (1) lediglich während eines Einsaugens durch einen Patienten in die erste Position zur Zerstäubung der Substanz bewegt.

7. Zerstäuber nach einem der Ansprüche 1 bis 5, gekennzeichnet durch einen Lufteinlaß (13) und einen Luftauslaß (14) für den Luftstrom, wobei der Luftauslaß (14) dazu dient, einen Fluß der zerstäubten Substanz zu einem Patienten zu ermöglichen.

8. Zerstäuber nach Anspruch 7, dadurch gekennzeichnet, daß sich die Ablenkvorrichtung (1) in die erste Position zur Zerstäubung bewegt, wenn der Luftstrom von dem Lufteinlaß (13) zu dem Luftauslaß (14) fließt.

9. Zerstäuber nach Anspruch 7 oder 8, dadurch gekennzeichnet, daß sich die Ablenkvorrichtung (1) in die zweite und nichtzerstäubende Position bewegt, wenn der Luftstrom unterbrochen wird.

10. Zerstäuber nach Ansprüchen 7 bis 9, dadurch gekennzeichnet, daß das Blatt (2) zu einer Position vorgespannt wird, bei welcher der Lufteinlaß (13) geschlossen wird.

11. Zerstäuber nach einem der Ansprüche 8 bis 10, dadurch gekennzeichnet, daß, wenn der Luftstrom von dem Lufteinlaß (13) zu dem Luftauslaß (14) fließt, das Blatt (2) Luft in den Bereich des Zerstäubers leitet, in welchem eine Zerstäubung stattfindet.

12. Zerstäuber nach Ansprüchen 10 und 11, dadurch gekennzeichnet, daß, wenn der Luftstrom von dem Luftauslaß (14) zu dem Lufteinlaß (13) fließt, das Blatt (2) es ermöglicht, daß Luft direkt von dem Luftauslaß (14) zu dem Lufteinlaß (13) fließt, ohne durch den Bereich des Zerstäubers hindurchzufließen, in welchem eine Zerstäubung stattfindet, wenn sich die Ablenkvorrichtung in der ersten Position befindet.

13. Zerstäuber nach einem der Ansprüche 7 bis 12, dadurch gekennzeichnet, daß, wenn der Luftstrom von dem Lufteinlaß (13) zu dem Luftauslaß (14) eine vorbestimmte Flußrate übersteigt, ein Anteil des Luftstroms den Bereich des Zerstäubers umgeht, in welchem eine Zerstäubung stattfindet, wenn sich die Ablenkvorrichtung in der ersten Position befindet.

14. Zerstäuber nach einem der vorhergehenden Ansprüche, dadurch gekennzeichnet, daß die Ablenkvorrichtung (1) eine Längs-Ablenkvorrichtung ist und seitlich bezüglich ihrer Länge zwischen ihrer ersten und zweiten Position beweglich ist.

15. Zerstäuber nach einem der Ansprüche 1 bis 13, dadurch gekennzeichnet, daß die Ablenkvorrichtung (1) gebogen ist und um die Achse dieses Bogens um einen Winkel verschiebbar ist.

16. Zerstäuber nach einem der vorhergehenden Ansprüche, dadurch gekennzeichnet, daß die Ablenkvorrichtung (1) eine Messerschneide umfaßt, welche bei der ersten Position dem Gas, welches aus dem Gasaustritt (4) austritt, dargeboten wird.

17. Zerstäuber nach einem der Ansprüche 1 bis 13, dadurch gekennzeichnet, daß die Ablenkvorrichtung (1) gebogen und um die Achse dieses Bogens um einen Winkel verschiebbar ist und eine Messerschneide umfaßt, welche bei der ersten Position dem Gas, welches aus dem Gasaustritt (4) austritt, dargeboten wird, und dadurch gekennzeichnet, daß die Ablenkvorrichtung in Längsrichtung bezüglich der Kante davon zwischen der ersten und zweiten Position beweglich ist.

18. Zerstäuber nach einem der vorhergehenden Ansprüche, wobei die Ablenkvorrichtung, wenn diese in ihre zweite Position bewegt wird, außerhalb des Weges des Gases, welches aus dem Gasaustritt austritt, liegt.

19. Zerstäuber nach einem der Ansprüche 1 bis 16, dadurch gekennzeichnet, daß die Ablenkung zu dem Gasausgang (4) hin und von diesem fort beweglich ist.

20. Zerstäuber nach Anspruch 19, dadurch gekennzeichnet, daß die Ablenkvorrichtung, wenn diese in ihre zweite Position bewegt wird, von dem Gasaustritt entfernt wird, so daß eine Zerstäubung nicht stattfindet.

21. Zerstäuber nach einem der Ansprüche 6 bis 20, dadurch gekennzeichnet, daß der Luftstrom durch das Atmungsmuster einer Person erzeugt wird.

22. Zerstäuber nach Anspruch 21, dadurch gekennzeichnet, daß die zerstäubte Substanz durch Luft mitgerissen wird, welche durch die Person eingeatmet wird.

23. Zerstäuber nach Anspruch 21 oder 22, dadurch gekennzeichnet, daß die durch die Person ausgeatmete Luft die zu zerstäubende Substanz nicht mitreißt.

24. Zerstäuber nach einem der vorhergehenden Ansprüche, dadurch gekennzeichnet, daß die zu zerstäubende Substanz ausgewählt ist aus: einer Flüssigkeit, einem pulverisierten Feststoff und einem Kolloid.

25. Zerstäuber nach einem der vorhergehenden Ansprüche, gekennzeichnet durch einen Düsenauslaß zum Erzeugen eines Sprühnebels aus der zu zerstäubenden Substanz.

26. Zerstäuber nach Anspruch 1 oder 2, wobei die Einrichtung zum Bewegen der Ablenkvorrichtung einen Luftstromsensor und eine Einrichtung umfaßt, welche auf den Sensor anspricht, um die Position der Ablenkvorrichtung zu ändern.

27. Zerstäuber nach einem der Ansprüche 1 bis 4, wobei die Ablenkvorrichtung eine Kugel ist.

28. Zerstäuber nach einem der vorhergehenden Ansprüche, ferner umfassend eine Prallplatte (9), welche nach außen und nach unten um den Gasaustritt (4) verläuft, wobei die Ablenkvorrichtung (1) innerhalb der Prallplatte (9) angebracht ist.

## Revendications

1. Atomiseur comportant une sortie de gaz (4), un orifice de sortie (5) adjacent à la sortie de gaz (4), et un déflecteur (1) pour dévier le gaz sortant de la sortie de gaz (4), ce déflecteur (1) étant déplaçable entre une première position qu'il occupe sur la trajectoire du gaz provenant de la sortie de gaz (4) et une seconde position, caractérisé en ce que le déflecteur (1) sert à faire dévier le gaz sortant de la sortie de gaz (4) vers l'orifice de sortie (5) afin d'évacuer une substance à atomiser par l'orifice de sortie (5) et d'atomiser la substance dans le gaz provenant de la sortie de gaz (4), en ce que la première position du déflecteur (1) permet l'atomisation, et en ce que la seconde position est une position de non-atomisation.

2. Atomiseur selon la revendication 1, caractérisé en ce qu'il comporte un moyen pour déplacer le déflecteur entre la première et la seconde position.

3. Atomiseur selon la revendication 2, caractérisé en ce que le moyen pour déplacer le déflecteur consiste en une aube (2) qui est déviée par le passage de l'air aspiré à travers l'atomiseur.

4. Atomiseur selon la revendication 3, caractérisé en ce que l'aube (2) est montée sur charnière pour un mouvement de pivotement.

5. Atomiseur selon l'une quelconque des revendications précédentes, caractérisé en ce que le déflecteur (1) est une barrette.

6. Atomiseur selon l'une quelconque des revendications précédentes, comprenant une arrivée d'air (13) et une sortie d'air (14) pour le passage de l'air transportant la substance atomisée vers un patient et dans lequel un déflecteur (1) se déplace dans la première position pour atomiser la substance uniquement pendant l'inspiration du patient.

7. Atomiseur selon l'une quelconque des revendications 1 à 5, caractérisé par une arrivée d'air (13) et une sortie d'air (14) pour le flux d'air, la sortie d'air (14) permettant au flux de la substance atomisée à se propager vers un patient.

8. Atomiseur selon la revendication 7, caractérisé en ce que le déflecteur (1) se met dans la première position pour l'atomisation lorsque le flux d'air passe de l'arrivée d'air (13) à la sortie d'air (14).

9. Atomiseur selon la revendication 7 ou 8, caractérisé en ce que le déflecteur (1) se déplace dans la seconde position pour la non-atomisation lorsque le flux d'air est interrompu.

10. Atomiseur selon les revendications 7 à 9, caractérisé en ce que l'aube (2) est décentrée vers une position obturant l'arrivée d'air (13).

11. Atomiseur selon l'une quelconque des revendications 8 à 10, caractérisé en ce que, lorsque le flux d'air passe de l'arrivée d'air (13) à la sortie d'air (14), l'aube (2) dirige de l'air dans la région de l'atomiseur dans laquelle a lieu l'atomisation.

12. Atomiseur selon les revendications 10 et 11, caractérisé en ce que lorsque le flux d'air passe de la sortie d'air (14) à l'arrivée d'air (13), l'aube (2) permet à l'air de circuler directement de la sortie d'air (14) à l'arrivée d'air (13) sans passer par l'endroit de l'atomiseur dans lequel a lieu l'atomisation lorsque le déflecteur est dans la première position.

13. Atomiseur selon l'une quelconque des revendications 7 à 12, caractérisé en ce que lorsque le flux d'air de l'arrivée d'air (13) à la sortie d'air (14) dépasse un débit prédéterminé, une proportion du flux d'air est déviée de la région de l'atomiseur dans laquelle a lieu l'atomisation lorsque le déflecteur est dans la première position.

14. Atomiseur selon l'une quelconque des revendications précédentes, caractérisé en ce que le déflecteur (1) est un déflecteur longitudinal, et est déplaçable latéralement par rapport à sa longueur entre sa première et sa seconde position.

15. Atomiseur selon l'une quelconque des revendications 1 à 13, caractérisé en ce que le déflecteur (1) est courbé en forme d'arc et angulairement déplaçable autour de l'axe de cet arc.

16. Atomiseur selon l'une quelconque des revendications précédentes, caractérisé en ce que le déflecteur (1) comporte un bord tranchant qui se présente dans la première position au gaz provenant de la sortie de gaz (4).

17. Atomiseur selon l'une quelconque des revendications 1 à 13, caractérisé en ce que le déflecteur (1) est incurvé en forme d'arc et angulairement déplaçable autour de l'axe de cet arc et comporte un bord tranchant qui se présente dans la première position face au gaz provenant de la sortie de gaz (4), et en ce que le déflecteur est déplaçable longitudinalement par rapport à son bord entre la première et la seconde position.

18. Atomiseur selon l'une quelconque des revendications précédentes, dans lequel le déflecteur, lorsqu'il est déplacé dans sa seconde position, est situé hors de la trajectoire du gaz provenant de la sortie de gaz.

19. Atomiseur selon l'une quelconque des revendications 1 à 16, caractérisé en ce que le déflecteur est déplaçable en rapprochement ou en éloignement de la sortie de gaz (4).

20. Atomiseur selon la revendication 19, caractérisé en ce que le déflecteur, lorsqu'il est déplacé dans sa seconde position, se trouve situé à distance de la sortie de gaz de façon à ce que l'atomisation n'ait pas lieu.

21. Atomiseur selon l'une quelconque des revendications 6 à 20, caractérisé en ce que le flux d'air est généré par la respiration d'une personne.

22. Atomiseur selon la revendication 21, caractérisé en ce que la substance atomisée est entraînée par l'air inhalé par la personne.

23. Atomiseur selon la revendication 21 ou 22, caractérisé en ce que l'air expiré par la personne n'entraîne pas la substance à atomiser.

24. Atomiseur selon l'une quelconque des revendications précédentes, caractérisé en ce que la substance à atomiser est choisie parmi un liquide, un solide pulvérisé et un colloïde.

25. Atomiseur selon l'une quelconque des revendications précédentes, caractérisé par un orifice de sortie en forme de gicleur générant une pulvérisation de la substance à atomiser.

26. Atomiseur selon la revendication 1 ou 2, dans lequel le moyen permettant de déplacer le déflecteur comprend un capteur de flux d'air et un moyen pour modifier la position du déflecteur réagissant au capteur.

27. Atomiseur selon l'une quelconque des revendications 1 à 4, dans lequel le déflecteur est une boule.

28. Atomiseur selon l'une quelconque des revendications précédentes, comprenant en outre une chicane (9) s'étendant à l'extérieur et vers le bas autour de la sortie du gaz (4), le déflecteur (1) étant monté à l'intérieur de la chicane (9).
